## Europäisches Patentamt
## European Patent Office
## Office européen des brevets

(11) Veröffentlichungsnummer : **0 515 400 B1**

(12) **EUROPÄISCHE PATENTSCHRIFT**

(45) Veröffentlichungstag der Patentschrift :
**10.11.93 Patentblatt 93/45**

(51) Int. Cl.⁵ : **C04B 35/66, C04B 35/04, C04B 35/42, C04B 35/48**

(21) Anmeldenummer : **91902940.5**

(22) Anmeldetag : **16.01.91**

(86) Internationale Anmeldenummer :
**PCT/EP91/00078**

(87) Internationale Veröffentlichungsnummer :
**WO 91/12217 22.08.91 Gazette 91/19**

(54) **MASSE ZUM BESCHICHTEN EINER AUSKLEIDUNG EINES METALLURGISCHEN SCHMELZGEFÄSSES AUF DER BASIS FEUERFESTER OXIDE.**

(30) Priorität : **16.02.90 DE 4004870**

(43) Veröffentlichungstag der Anmeldung :
**02.12.92 Patentblatt 92/49**

(45) Bekanntmachung des Hinweises auf die Patenterteilung :
**10.11.93 Patentblatt 93/45**

(84) Benannte Vertragsstaaten :
**AT DK ES LU NL SE**

(56) Entgegenhaltungen :
**EP-A- 187 338**
**EP-A- 253 957**
**GB-A- 2 196 331**
**WORLD PATENT INDEX, DERWENT PUBLICATIONS LTD, LONDON, GB, ACCESSION NUMBER 80-91003C (WEEK 8051) & JP55140758 (AIKOH KK) (05/11/1980)**
**WORLD PATENTS INDEX (LATEST) DERWENT PUBLICATIONS LTD., LONDON, GB. ACCESSION NUMBER 89-373462 (WEEK 8951) & JP1278469 (KAWASAKI ROZAI KK) (08/11/1989)**
**WORLD PATENTS INDEX (LATEST) DERWENT PUBLICATIONS LTD., LONDON, GB. ACCESSION NUMBER 87-077701 (WEEK 8711) & SU1244131 (AS USSR HIGH TEMP INST) (15/07/1986)**
**CHEMICAL ABSTRACTS, vol. 97, No. 22, 29 November 1982, Columbus, Ohio, USA "BASIC CASTABLE REFRACTORIES" ref. no. 187074V, see abstract**

(56) Entgegenhaltungen :
**CHEMICAL ABSTRACTS, vol. 101, No. 10, September 1984, Columbus, Ohio, USA, "MAGNESIA-CHROME CASTABLE REFRACTORIES" PAGE 305; right-hand column; ref. no. 77725B, see abstract**

(73) Patentinhaber : **VEITSCH-RADEX AKTIENGESELLSCHAFT FÜR FEUERFESTE ERZEUGNISSE**
**Mommsengasse 35**
**A-1040 Wien (AT)**

(72) Erfinder : **MÖRTL, Günter**
**Hammergasse 20**
**A-9500 Villach (AT)**
Erfinder : **Siegl, Walter**
**Enzianweg 2**
**A-9500 Villach (AT)**
Erfinder : **LOOP, Peter**
**Wüstenrotstrasse 5/1**
**A-9500 Villach (AT)**

(74) Vertreter : **Becker, Thomas, Dr. et al**
**Becker & Müller Patentanwälte**
**Eisenhüttenstrasse 2**
**D-40882 Ratingen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Masse zum Beschichten einer Auskleidung eines metallurgischen Schmelzgefäßes auf der Basis feuerfester Oxide wie Sintermagnesia, Chrommagnesia, Sinterdolomit, Chromerz und/oder Zirkondioxid.

Eine solche Masse auf Basis Sintermagnesit und/oder Sinterdolomit ist aus der DE-PS 37 03 136 bekannt. Die Masse enthält zusätzlich 0,5 bis 5 Gew.-% Bentonit, 1 bis 5 Gew.-% Aluminiumsulfat und 0,5 bis 5 Gew.-% einer Erdalkaliverbindung in Form von MgO-Kauster und/oder Magnesiumhydroxid.

Die bekannte Masse soll beim Aufspritzen auf heiße Flächen von Öfen rasch abbinden und eine hohe Feuerfestigkeit besitzen.

Eine ähnliche Masse ist auch aus der DE-PS 37 39 900 bekannt, wobei als feuerfeste Oxide neben Sinter- oder Schmelzmagnesit 10 bis 25 Gew.-% einer Zirkoniumverbindung mit einem $ZrO_2$-Gehalt von wenigstens 98 Gew.-%, bezogen auf das MgO vorgeschlagen wird. Mit dieser Masse soll ebenfalls in erster Linie eine hohe Temperaturbeständigkeit bis zu Temperaturen oberhalb von 1900° C erreicht werden.

Aus World Patent Index, Derwent Publications, Accession Number 80-91003 C (week 8051) ist eine Tundish-Masse bekannt, die 70 bis 90 Gew.-% Magnesit-Klinker (MgO) und 1 bis 10 Gew.-% Magnesiumcarbonat ($MgCO_3$) aufweist.

In Chemical Abstracts, vol. 101 (1984) No. 10 ref.no. 77725b sind feuerfeste Werkstoffe auf MgO-Basis beschrieben, die Chromerz und Aluminat-Zement enthalten. Die chemische Analyse weist 8 Gew.-% $CaCO_3$ auf.

Die bekannten, gattungsgemäßen Massen weisen einen relativ geringen Gehalt an CaO auf, der insbesondere bei Verwendung von Sinterdolomit eingebracht wird. Metallurgisch sind aber kalziumoxidreiche Überzugsmassen in Bezug auf die Stahlreinheit und Abscheidung nichtmetallischer Einschlüsse durchaus erstrebenswert. Höhere Anteile an Kalziumoxid scheiden jedoch stets dann aus, wenn die Masse mit Wasser aufbereitet wird, weil das Kalziumoxid dann mit dem Wasser spontan zu Kalziumhydroxid reagiert, was die Verarbeitbarkeit verschlechtert oder unmöglich macht.

Der Erfindung liegt insoweit die Aufgabe zugrunde, eine gattungsgemäße Masse anzubieten, die einen möglichst hohen Gehalt an Kalziumoxid aufweist und trotzdem naß verarbeitet werden kann.

Der Erfindung liegt die Erkenntnis zugrunde, daß dieses Ziel überraschend einfach durch eine gattungsgemäße Masse erreicht werden kann, die 10 bis 50 Gew.-%, bezogen auf die Gesamtmasse, an karbonatisch gebundenem Kalzium- und/oder Magnesiumoxid in Form von Dolomit ($MgCa(CO_3)_2$) oder Kalk ($CaCO_3$) enthält.

Der bevorzugte Gehalt liegt zwischen 10 und 40 Gew.-%, bezogen auf die Gesamtmasse, wobei Calciumcarbonat gegenüber Magnesiumcarbonat wegen seiner günstigen metallurgischen Wirkung Vorteile besitzt.

Die Verwendung von Karbonaten innerhalb der Masse ist zunächst überraschend, weil das Karbonat bei der sich üblicherweise an das Aufbringen der Masse auf die Auskleidung eines metallurgischen Schmelzgefäßes anschließenden Wärmebehandlung (Aufheizung) beziehungsweise der anschließenden Erwärmung durch die Metallschmelze zwangsweise zersetzt und damit die Festigkeit herabsetzt. Es wäre deshalb zu vermuten, daß durch die Zersetzung des Karbonats die Festigkeit der monolithischen Masse insbesondere bei höheren Gehalten an karbonatisch gebundenem CaO (und MgO) deutlich abnimmt und so die Standsicherheit der Beschichtung gefährdet. Überraschenderweise hat sich jedoch herausgestellt, daß feuerseitig (auf der der Schmelze zugewandten Seite) die Temperaturen der Metallschmelze so hoch sind, daß es gleichzeitig zu einer ausreichenden Sinterung und damit zu einer Verfestigung der Oberflächenschicht der monolithischen Masse kommt, die ihre Feuerfestigkeit und Stabilität allenfalls unerheblich verringert. Dem gegenüber wirkt sich die Zersetzung des Karbonates nur in dem Bereich der monolithischen Beschichtung festigkeitsmindernd aus, die der Metallschmelze abgewandt beziehungsweise dem Dauerfutter zugewandt ist. Der Grund liegt darin, daß in diesem Bereich die Temperaturen zwangsläufig geringer sind als auf der "Feuerseite", so daß hier keine Versinterung in dem Maße eintritt wie auf der der Schmelze Zugewandten Seite.

Vielmehr bleibt der entsprechende Teil der Masse mürbe, selbst wenn der der Metallschmelze zugewandte Abschnitt voll durchgesintert ist.

Dieses Phänomen ist jedoch bei speziellen Anwendungen durchaus erwünscht, wo ein fester Verbund zwischen der Überzugsmasse und dem Dauerfutter verhindert werden soll. Hierdurch wird es nämlich ermöglicht, die nur einmal verwendete monolithische Auskleidung bei deren Erneuerung leicht vom Dauerfutter des metallurgischen Schmelzgefäßes zu lösen und mit dem Metallbären problemfrei auszukippen. Ein weiterer Vorteil der Zersetzung des Karbonats liegt darin, daß gleichzeitig die Porosität der Masse zunimmt, wodurch die thermische Isolierung verbessert wird.

Vor allem aber besitzt die erfindungsgemäße Masse den entscheidenden Vorteil, daß sie reich an Kalziumoxid ist und dadurch die oben beschriebenen metallurgischen Vorteile ermöglicht.

In einer vorteilhaften Ausführungsform der Erfindung wird vorgeschlagen, der Masse ein anorganisches Bindemittel, beispielsweise Wasserglas, zuzusetzen, und zwar vorzugsweise in einer Menge von 1 bis 3 Gew.-%, bezogen auf die Gesamtmasse. Ebenso kann aber auch ein organisches Bindemittel wie Phenolnovolak oder Trockenbinderit zugegeben werden, und zwar vorteilhafterweise ebenfalls in einer Menge bis zu 5 Gew.-%, bezogen auf die Gesamtmasse.

Darüber hinaus können der Masse übliche Zusätze der nachfolgend genannten Art zugegeben werden, wobei die Gewichtsangaben sich jeweils auf die Gesamtmasse beziehen:
- anorganische und/oder organische Faserstoffe (bis zu 5 Gew.-%)
- anorganische und/oder organische plastifizierende Zusätze (zum Beispiel Ton, Polyvinylalkohol etc.) (bis zu 5 Gew.-%).
- luftporenbildende und/oder verflüssigende Zusätze (bis zu 1,0 Gew.-%)

Die erfindungsgemäße Masse kann trocken und naß aufbereitet werde. Als Trockenmasse wird sie hinter eine entsprechende Schablone durch Vibration eingebracht und dort unter Wärmeeinwirkung bei Temperaturen über 200° C ausgehärtet.

Besonders vorteilhaft läßt sich die erfindungsgemäße Masse auch mit Wasser anmischen und zum Beispiel aufschmieren oder aufspritzen (Torkretieren). Dabei kann die Masse - insbesondere unter Verwendung luftporenbildender oder verflüssigender Zusätze - zu einem luftporenreichen Schlamm mit Wasser angemischt und mittels Pumpspritzverfahren auf die Auskleidung des metallurgischen Schmelzgefäßes aufgebracht werden.

Die Masse kann besonders vorteilhaft unter anderem als Tundish-Überzugsmasse eingesetzt werden.

Weitere Merkmale der Erfindung ergeben sich aus den Merkmalen der Unteransprüche.

Die nachfolgenden Beispiele verdeutlichen die beschriebenen Eigenschaften und Vorteile.

Versuchsreihe I zeigt eine konventionelle Spritzmasse ohne $CaCO_3$-Anteil (A) im Vergleich zu zwei erfindungsgemäßen Spritzmassen mit 20 Gew.-% $CaCO_3$ (B) beziehungsweise 30 Gew.-% $CaMg(CO_3)_2$ (C). In allen Fällen liegen die Massen in einer Kornverteilung mit 98 Gew.-% kleiner 2 mm vor.

Die nachfolgende Tabelle zeigt im einzelnen die Rohstoffbasis, chemische Analyse, Porosität und Kaltdruckfestigkeit bei verschiedenen Temperaturen sowie Haftung der Masse am Dauerfutter nach Gießende.

Versuchsreihe I (alle Angaben in Gewichtsprozent, falls nicht anders angegeben)

| Spritzmasse | | A | B | C |
|---|---|---|---|---|
| **Rohstoffbasis** | | | | |
| Sintermagnesia | | 96,3 | 76,3 | 66,3 |
| $CaCO_3$ | | - | 20 | - |
| $CaMg (CO_3)_2$ | | - | - | 30 |
| Wasserglas (als anorganisches Bindemittel) | | 3 | 3 | 3 |
| Zellulosefasern | | 0,5 | 0,5 | 0,5 |
| Methylcellulose | | 0,2 | 0,2 | 0,2 |
| chemische Analyse | MgO | 83,2 | 68,7 | 67,2 |
| | $Fe_2O_3$ | 4,9 | 4,0 | 4,0 |
| | $Al_2O_3$ | 0,4 | 0,4 | 0,3 |
| | CaO | 5,9 | 15,5 | 13,8 |
| | $SiO_2$ | 2,8 | 2,7 | 2,8 |
| | Glühverlust | 0,9 | 7,2 | 10,2 |
| Porosität | 110° C | 35,3 | 34,7 | 36,0 |
| (Vol.-%) bei | 1000° C | 39,2 | 40,1 | 44,0 |
| | 1400° C | 34,7 | 40,6 | 43,6 |
| | 1550° C | 32,5 | 37,8 | 38,1 |
| Kaltdruckfestig- | 110° C | 4,3 | 3,9 | 3,0 |
| keit (N/mm2) bei | 1000° C | 2,0 | 1,6 | 0,7 |
| | 1400° C | 13,1 | 2,2 | 1,2 |
| | 1550° C | 31,7 | 23,4 | 21,9 |
| Haftung am Dauerfutter nach Gießende | | ++ / + | + / 0 | 0 |

++ stark          +  schwach          0  keine Haftung

Entsprechend der Rohstoffbasis ist der CaO-Gehalt der Spritzmassen B und C deutlich höher gegenüber der Spritzmasse A.

Deutlich ist auch zu erkennen, daß die Porosität im Temperaturbereich zwischen 1000° C und 1400° C (also auf der "kalten" Seite der fertig aufgebrachten Masse) bei den Versätzen nach B und C deutlich höher ist als bei der Masse A, woraus die bereits vorstehend genannte verbesserte thermische Isolierung resultiert.

Die Werte der Kaltdruckfestigkeit zeigen ferner, daß diese bei den erfindungsgemäßen Massen zunächst mit steigender Temperatur deutlich sinkt, und zwar bis zu Temperaturen von etwa 1400° C. Daraus folgt, daß die erfindungsgemäßen Massen auf ihrer "kalten", also der Metallschmelze abgewandten Seite, praktisch keine eigene Festigkeit besitzen und "mürbe" bleiben, während sie auf der "Feuerseite", also bei Temperaturen oberhalb 1500° C durch Versinterung wieder eine hohe Festigkeit erlangen. Aus diesem "Phänomen" leitet sich ab, daß die erfindungsgemäßen Massen nur eine schwache oder überhaupt keine Haftung gegenüber dem Dauerfutter (also an der "kalten" Seite) besitzen mit dem daraus resultierenden Vorteil, daß sich die Masse nach dem Einsatz leicht ablösen läßt.

In der Versuchsreihe II ist eine konventionelle Schlämmespritzmasse (D) einer erfindungsgemäßen Schlämmespritzmasse (E) mit einem Anteil von 25 Gew.-% $CaCO_3$ gegenübergestellt.

Die anhand der Versuchsreihe I beschriebenen Eigenschaften und Vorteile der erfindungsgemäßen Massen werden durch die angegebenen Versuchsergebnisse auch hier bestätigt.

## Versuchsreihe II (alle Angaben in Gewichtsprozent, falls nicht anders angegeben)

| Schlämmespritzmasse | | D | E |
|---|---|---|---|
| **Rohstoffbasis** | | | |
| Sintermagnesia | | 96,7 | 71,7 |
| $CaCO_3$ | | - | 25 |
| Wasserglas (als anorganisches Bindemittel) | | + 2,5 | + 2,5 |
| Zellulosefasern | | + 0,5 | + 0,5 |
| Luftporenbildende Zusätze | | + 0,3 | + 0,3 |
| chemische Analyse | MgO | 88,0 | 68,4 |
| | $Fe_2O_3$ | 1,0 | 0,9 |
| | $Al_2O_3$ | 1,5 | 1,4 |
| | CaO | 1,4 | 14,5 |
| | $SiO_2$ | 5,2 | 4,6 |
| | Glühverlust | 1,5 | 9,0 |
| Porosität | 110° C | 52,2 | 51,8 |
| (Vol.-%) bei | 1000° C | 54,6 | 55,0 |
| | 1400° C | 51,7 | 57,1 |
| | 1550° C | 48,2 | 54,6 |
| Kaltdruckfestig- | 110° C | 1,8 | 1,5 |
| keit (N/mm2) bei | 1000° C | 1,6 | 1,4 |
| | 1400° C | 6,8 | 1,1 |
| | 1550° C | 23,4 | 18,7 |
| Haftung am Dauerfutter nach Gießende | | +/0 | 0 |

++ stark          +   schwach          0   keine Haftung

## Patentansprüche

1. Masse zum Beschichten einer Auskleidung eines metallurgischen Schmelzgefäßes auf der Basis von Sintermagnesia, Chrommagnesia, Sinterdolomit, Chromerz und/oder Zirkondioxid, dadurch gekennzeichnet, daß sie 10 bis 50 Gew.-% $CaCO_3$ und gegebenenfalls $MgCO_3$, bezogen auf die Gesamtmasse, in Form von Dolomit oder Kalk enthält.

2. Masse nach Anspruch 1, dadurch gekennzeichnet, daß der Gehalt an $CaCO_3$ und/oder $MgCO_3$ zwischen 10 und 40 Gew.-% liegt.

3. Masse nach Anspruch 1 oder 2, dadurch gekennzeichnet, daß sie in einer Körnung kleiner 2 mm konfektioniert ist.

4. Masse nach einem der Ansprüche 1 bis 3, dadurch gekennzeichnet, daß sie ein anorganisches Bindemittel, vorzugsweise Wasserglas, in einer Menge von 1 bis 3 Gew.-%, bezogen auf die Gesamtmasse, enthält.

5. Masse nach einem der Ansprüche 1 bis 4, dadurch gekennzeichnet, daß sie ein organisches Bindemittel wie Phenolnovolak in einer Menge bis zu 5 Gew.-%, bezogen auf die Gesamtmasse, enthält.

6. Verfahren zum Aufbringen einer feuerfesten Masse nach einem der Ansprüche 1 bis 5 auf einer Auskleidung eines metallurgischen Schmelzgefäßes, dadurch gekennzeichnet, daß die Masse trocken hinter eine korrespondierende Schablone durch Vibration eingebracht oder nach Anmachen mit Wasser aufgeschmiert oder aufgespritzt, danach gegebenenfalls unter Wärmeeinwirkung bei Temperaturen überhalb 200° C ausgehärtet wird, bevor eine zweite Masse bekannter Zusammensetzung (ohne einen Gehalt an Karbonaten) auf die zuvor aufgetragene Schicht aufgebracht wird.

## Claims

1. Composition based on sintered magnesia, chrome magnesia, sintered dolomite, chromium ore, and/or zirconium dioxide for coating a lining of a metallurgical melting vessel, characterized in that it contains 10 to 50 wt.% $CaCO_3$ and/or $MgCO_3$ in the form of dolomite or lime relative to the total composition.

2. Composition in accordance with claim 1, characterized in that the $CaCO_3$ and/or $MgCO_3$ content is between 10 and 40 wt.%.

3. Composition in accordance with claim 1 or 2, characterized in that it is prepared with a particle size of less than 2 mm.

4. Composition in accordance with one of the claims 1 through 3, characterized in that it contains an inorganic binder, preferably water glass, in an amount of 1 to 3 wt.% relative to the total composition.

5. Composition in accordance with one of the claims 1 through 4, characterized in that it contains an organic binder, such as phenol novolak, in an amount of up to 5 wt.% relative to the total composition.

6. Process for applying a refractory composition in accordance with one of the claims 1 through 5 to a lining of a metallurgical melting vesssel, characterized in that in the dry state, the composition is introduced by vibration behind a corresponding template, or is spread or sprayed after mixing with water, after which it is hardened, if desired, by the effect of heat at temperatures above 200°C, before a second composition of prior-art composition (containing no carbonates) is applied to the layer applied previously.

## Revendications

1. Matière pour couvrir le revêtement d'un bassin de fusion métallurgique à base de magnésie frittée, la magnsie-chromite, la dolomie frittée, le minerai de chrome et/ou le dioxyde de zirconium, caractérisée en ce qu'elle contient 10 à 50 % en poids de $CaCO_3$ et le cas échéant, de $MgCO_3$, par rapport à la matière finale, sous forme de dolomie ou de chaux.

2. Matière selon la revendication 1, caractérisée en ce que la teneur en $CaCO_3$ et/ou $MgCO_3$ est comprise entre 10 et 40 % en poids.

3. Matière selon la revendication 1 ou 2, caractérisée en ce qu'elle est constituée par des grains de granulation inférieure à 2mm.

4.  Matière selon l'une des revendications 1 à 3, caractérisée en ce qu'elle contient un liant inorganique, de préférence un silicate soluble, dont la teneur est comprise entre 1 et 3 % en poids par rapport à la matière finale.

5.  Matière selon l'une des revendications 1 à 4, caractérisée en ce qu'elle contient un liant organique, tel que le novolaquephénolique dont la teneur peut atteindre 5 % en poids par rapport à la matière finale.

6.  Procédé pour épandre une matière réfractaire selon l'une des revendications 1 à 5 sur le revêtement d'un bassin de fusion métallurgique, caractérisé en ce qu'on place sous vibrations la matière sèche dans un moule conforme ou bien on l'étale après gâchage à l'eau ou on l'applique au pistolet, puis on cuit par un traitement à chaud à une température supérieure à 200° C, avant d'appliquer une seconde matière de composition connue sans un teneur de carbonates sur la couche préalablement déposée.